# EUROPEAN PATENT APPLICATION

(11) **EP 1 964 508 A2**
(43) Date of publication of application: **03.09.2008**
(21) Application number: 08010747.7
(22) Date of filing: 10.06.2005
(51) Int. Cl.: A61B 1/00, A61B 1/01, A61M 25/00, A61B 1/12

(54) **Endoscope apparatus**

(30) Priority: 14.06.2004 JP 2004175647; 05.11.2004 JP 2004322794; 24.03.2005 JP 2005086893
(62) Divisional of application: 05012569.9
(71) Applicant: Fujinon Corporation, Saitama-shi, Saitama (JP); SRJ Corporation, Minami-Kawachi-machi Kawachi-gun Tochigi (JP)
(72) Inventor: Takano, Masayuki, Saitama-shi, Saitama (JP); Fujikura, Tetsuya, Saitama-shi, Saitama (JP)
(74) Representative: Hering, Hartmut

(57) **Abstract**

An endoscope apparatus provided with an insertion aid (50) through whose base end of an insert part (12) of the endoscope (10) is inserted and whose tip (58) is fitted with a balloon (30), the balloon (30) being closely adhered to a body cavity by being inflated, wherein the outer circumferential face of the insertion aid (50) is coated with a lubricating coat material, and the friction coefficient of the outer circumferential face of the insertion aid (50) is set to 5 to 30% of the friction coefficient of the balloon (30).

## Description

The present invention relates to an endoscope apparatus having an endoscope to the tip of whose insert part balloons are fitted and an insertion aid for guiding the insert part of this endoscope into a body cavity.

When the insert part of an endoscope is to be inserted into a deep digestive canal, such as the small intestine, it will be difficult to be inserted deep enough if the insert part is merely pressed in because the complex bends of the intestine would prevent a strong enough force from being transmitted to the tip of the insert part. On account of this problem, it is proposed to fit the insert part of an endoscope with an insertion aid called an over-tube or a sliding tube before it is inserted into a body cavity, so that the insert part can be prevented from being unnecessarily bent or loosened by guiding the insert part with this insertion aid (e.g. Japanese Patent Application Laid Open No. 10-248794).

Conventional endoscope apparatuses include a double-balloon type, which has a first balloon at the tip of the insert part of the endoscope and a second balloon at the tip of the insertion aid (e.g. Japanese Patent Application Laid Open Nos. 2001-340462 and 2002-301019).

When using a double-balloon type endoscope apparatus, the insert part and the insertion aid are inserted by a prescribed length, and the winding intestinal tract may be contracted to a straight shape by hauling in the insert part and the insertion aid at the same time in a state in which the first and second balloons are inflated and stuck to the intestinal wall. After that, the intestinal tract is drawn to bring the insert part to its target region by sequentially repeating the insertion of the insert part, that of the insertion aid and their simultaneous hauling-in.

This operation is considerably affected by the friction between the insert part of the endoscope and the insertion aid because they move back and forth relative to each other. This problem is conventionally addressed by uniformly laying a lubricating coat, such as a hydrophilic coat, over the inner circumferential face of the insertion aid to reduce the frictional resistance between the outer circumferential face of the insert part of the endoscope and the inner circumferential face of the insertion aid, thereby to improve the insertion ease of the insert part of the endoscope. Also, a lubricating coat, such as a hydrophilic coat, is uniformly laid over the outer circumferential face of the insertion aid to reduce the frictional resistance between the insertion aid and the intestinal wall and thereby to facilitate insertion of the insertion aid.

However, since the insertion aid of the conventional endoscope apparatus described above is made uniformly hard from its base to top, it is difficult to sufficiently transmit the pressing force to the tip of the insertion aid when it is to be inserted into the winding intestinal tract (in vivo), resulting in a problem of difficulty to insert the insertion aid. There is another disadvantage that, since the inner circumferential face of the tip of the insertion aid is subject to high resistance from the insert part of the endoscope caused by the in-and-out movement of the insert part of the endoscope, the hydrophilic coat over the inner circumferential face of the tip becomes worn out soon.

An object of the present invention, attempted in view of these problems, is to provide an endoscope apparatus whose insertion aid can be inserted into a living body more easily and which enables the lubricating coats laid over its insertion aid and insert part of the endoscope to wear longer and to remain effective for a long period.

In order to achieve the object of the invention stated above, according to a first aspect of the invention, in an endoscope apparatus provided with an insertion aid through whose base end an insert part of the endoscope is inserted and whose tip is fitted with a balloon, the balloon being closely adhered to a body cavity by being inflated, the outer circumferential face of the insertion aid is coated with a lubricating coat material, and the friction coefficient of the outer circumferential face of the insertion aid is set to 5 to 30% of the friction coefficient of the balloon.

According to the first aspect of the invention, the frictional resistance to the wall of the body cavity is reduced by coating the outer circumferential face of the insertion aid with a lubricating coat material, and at the same time an appropriate level of frictional resistance is thereby prescribed for the balloon for which a relatively high level of frictional resistance is secured. Thus, the frictional resistance of the outer circumferential face of the insertion aid coated with the lubricating material is set to be 5 to 30% of the frictional resistance of the balloon. If the proportion of that frictional resistance is less than 5%, the insertion aid will prove too slippery in the hand of the operator manipulating it, and this might invite mishandling. Or if the proportion is more than 30%, it will become difficult to insert or withdraw the insertion aid against the wall of the body cavity. Therefore, the invention under the present application according to which the proportion of frictional resistance is set between 5 and 30% enables the insertion aid to be inserted and withdrawn into and out of the body cavity with satisfactory ease.

According to a second aspect of the invention, in an endoscope apparatus provided with an endoscope the tip of whose insert part is fitted with a balloon, the balloon being closely adhered to a body cavity by being inflated, the outer circumferential face of the insert part of the endoscope is coated with a lubricating coat material, and the friction coefficient of the outer circumferential face of the insert part is set to 5 to 30% of the friction coefficient of the balloon.

According to the second aspect of the invention, the frictional resistance to the wall of the body cavity is reduced by coating the outer circumferential face of the insert part of the endoscope with a lubricating coat material, and at the same time an appropriate level of frictional resistance is thereby prescribed for the balloon for which a relatively high level of frictional resistance is secured. Thus, the frictional resistance of the outer circumferential face of the insert part of the endoscope coated with the lubricating material is set to be 5 to 30% of the frictional resistance of the balloon. If the proportion of that frictional resistance is less than 5%, the insert part of the endoscope will prove too slippery in the hand of the operator manipulating the insertion aid, and this might invite mishandling. Or if the proportion is more than 30%, it will become difficult to insert or withdraw the insert part of the endoscope against the wall of the body cavity. Therefore, the invention under the present application according to which the proportion of frictional resistance is set between 5 and 30% enables the insert part of the endoscope to be inserted and withdrawn into and out of the body cavity with satisfactory ease.

In order to achieve the object stated above, according to a third aspect of the invention, there is provided an endoscope apparatus provided with an endoscope the tip of whose insert part is fitted with a first balloon and an insertion aid which covers the insert part to guide the insertion of the insert part and whose tip is fitted with a second balloon, the first balloon and the second balloon being closely adhered to a body cavity by being inflated, wherein the outer circumferential face of the insertion aid is coated with a lubricating coat material, and the friction coefficient of the outer circumferential face of the insertion aid is set to 5 to 30% of the friction coefficient of the second balloon.

The third aspect of the invention concerns an endoscope apparatus provided with an endoscope the tip of whose insert part is fitted with a first balloon and an insertion aid whose tip is fitted with a second balloon. In this endoscope apparatus, the friction coefficient of the outer circumferential face, coated with a lubricating coat material, of the insertion aid is set to 5 to 30% of the friction coefficient of the second balloon, coated with no lubricating coat material.

Since an endoscope apparatus having a first balloon and a second balloon is frequently pressed into a body cavity, it is essential to reduce the frictional resistance of the outer circumferential face of the insertion aid which is in contact with the wall of the body cavity it enters into. On the other hand, it is no less important to secure friction with the wall of the body cavity to ensure a sufficient holding force when the second balloon is inflated and adheres closely to the wall of the body cavity.

In view of this background, the frictional resistance to the wall of the body cavity is reduced, and at the same time an appropriate level of frictional resistance is prescribed for the second balloon for which a relatively high level of frictional resistance is secured, by coating the outer circumferential face of the insertion aid with the lubricating material. Thus, the frictional resistance of the outer circumferential face of the insertion aid coated with the lubricating material is set to be 5 to 30% of the frictional resistance of the second balloon. If the proportion of that frictional resistance is less than 5%, the insertion aid will prove too slippery in the hand of the operator manipulating it, and this might invite mishandling. Or if the proportion is more than 30%, it will become difficult to insert or withdraw the insertion aid against the wall of the body cavity. Therefore, the invention under the present application according to which the proportion of frictional resistance is set between 5 and 30% enables the insertion aid to be inserted and withdrawn into and out of the body cavity with satisfactory ease.

According to a fourth aspect of the invention, in the endoscope apparatus according to the third aspect, the outer circumferential face of the insert part of the endoscope is coated with a lubricating coat material, and the friction coefficient of the outer circumferential face of the coated insert part is set to 5 to 30% of the friction coefficient of the first balloon.

According to the fourth aspect of the invention, the frictional resistance to the wall of the body cavity is reduced, and at the same time an appropriate level of frictional resistance is prescribed for the balloon for which a relatively high level of frictional resistance is secured, by coating the outer circumferential face of the insert part of the endoscope with the lubricating material. Thus, the frictional resistance of the outer circumferential face of the insertion aid coated with the lubricating material is set to be 5 to 30% of the frictional resistance of the balloon. If the proportion of that frictional resistance is less than 5%, the insertion aid will prove too slippery in the hand of the operator manipulating it, and this might invite mishandling. Or if the proportion is more than 30%, it will become difficult to insert or withdraw the insertion aid against the wall of the body cavity. Therefore, the invention under the present application according to which the proportion of frictional resistance is set between 5 and 30% enables the insert part of the endoscope to be inserted and withdrawn into and out of the body cavity with satisfactory ease.

In an endoscope apparatus according to the invention provided with a first balloon and a second balloon, the friction coefficient of the outer circumferential face, coated with a lubricating coat material, of the insertion aid is set to 5 to 30% of the friction coefficient of the second balloon, enabling the insertion aid to be inserted and withdrawn into and out of the body cavity with satisfactory ease.

### BRIEF DESCRIPTION OF THE DRAWINGS

Fig. 1 shows the system configuration of an endoscope apparatus according to the present invention;
Fig. 2 shows a perspective view of the tip of the insert part of the endoscope;
Fig. 3 shows a perspective view of the tip of the insert part fitted with a first balloon;
Fig. 4 is a longitudinal section of an over-tube;
Figs. 5A through 5H illustrate how the endoscope apparatus according to the invention is operated;
Fig. 6 is a sectional view showing the thickness of the base and that of the hydrophilic coat of a first example of over-tube;
Fig. 7 is a sectional view showing the thickness of the base and that of the hydrophilic coat of a second example of over-tube;
Fig. 8 is a partial sectional view of an example in which the thickness of the hydrophilic coat of the insert part is gradually increased from the base end toward the tip; and
Fig. 9 is a partial sectional view of an example in which the thickness of the hydrophilic coat is increased only in the tip portion.

Preferred embodiments of the present invention will be described in detail below with reference to the accompanying drawings.

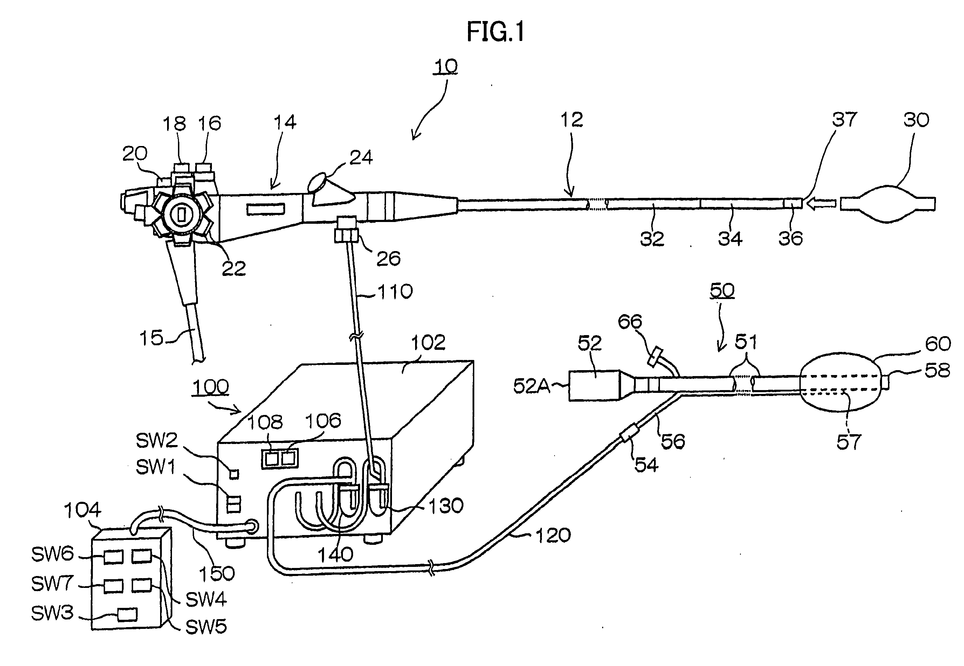

Fig. 1 shows the system configuration of a double-balloon type endoscope apparatus according to the invention. The double-balloon type endoscope apparatus shown here comprises an endoscope 10, an over-tube (insertion aid) 50 and a balloon control unit 100.

The endoscope 10 is equipped with a manipulating part 14 and an insert part 12 linked to this manipulating part 14. A universal cable 15 is connected to the manipulating part 14, and a processor and a connector (neither shown) connected to a light source unit are provided at the tip of the universal cable 15.

An air/water feed button 16, a suction button 18 and a shutter button 20, to be manipulated by the operator, are arranged collectively on the manipulating part 14, whereon a pair of angle knobs 22 and 22 and a forceps insert portion 24 are disposed in their respectively prescribed positions. The manipulating part 14 is further provided with a balloon air supply port 26 for supplying air to a first balloon 30 and sucking air from the balloon 30.

The insert part 12 comprises a soft portion 32, a bendable portion 34 and a tip 36. The bendable portion 34, composed by linking a plurality of nodule rings in a bendable way, is remotely bent by turning the pair of angle knobs 22 and 22 disposed on the manipulating part 14. The tip face 37 of the tip 36 can be thereby turned in a desired direction.

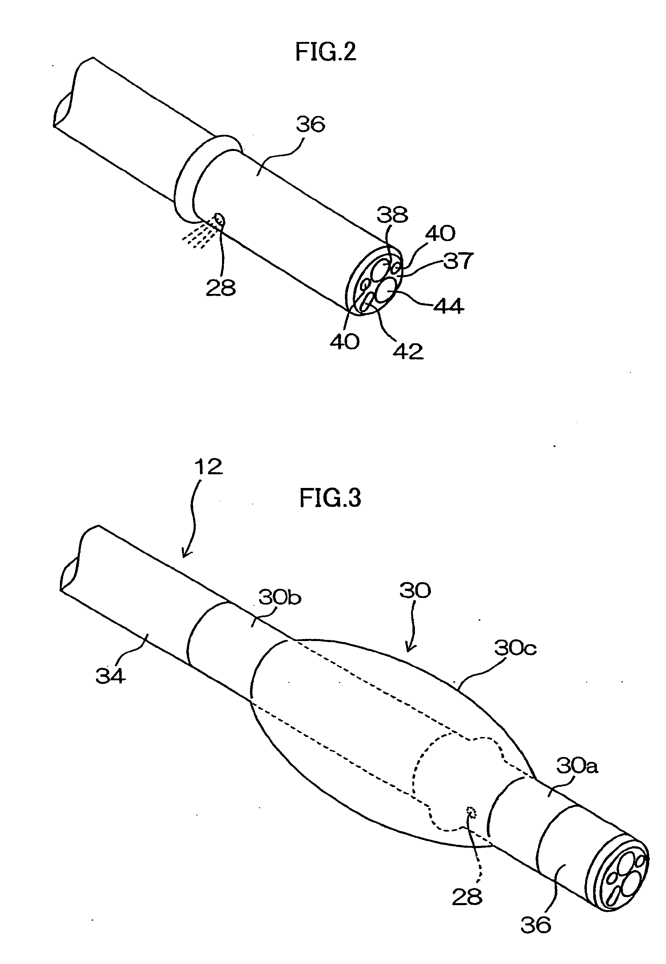

As shown in Fig. 2, an objective optical system 38, a pair of illuminating lenses 40 and 40, an air/water feed nozzle 42, a forceps inlet 44 and so forth are provided in respectively prescribed positions of the tip face 37 of the tip 36. An air supply intake 28 is provided in the outer circumferential face of the tip 36 as shown in Fig. 2 and Fig. 3, and this air supply intake 28 communicates with the balloon air supply port 26 shown in Fig. 1 via an air supply tube (not shown) inserted in the insert part 12. Therefore, by supplying air into the balloon air supply port 26, air is blown out of the air supply intake 28 in the tip 36, while air is sucked by way of the air supply intake 28 by sucking air through the balloon air supply port 26.

As shown in Fig. 1, the first balloon 30 consisting of an elastic material, such as rubber, is detachably fitted to the tip 36 of the insert part 12. The first balloon 30, formed of a bulged portion 30c in the middle and fitting portions 30a and 30b on its two sides as shown in Fig. 3, is so fitted to the tip 36 that the air supply intake 28 be positioned within the bulged portion 30c. The fitting portions 30a and 30b are formed to be smaller in diameter than the tip 36 and, after being tightly adhered to the tip 36 by their elastic forces, fixed by winding a thread (not shown) around them. Incidentally, the fixing method is not limited to winding a thread, but the fitting portions 30a and 30b can as well be fixed to the tip 36 by fitting a fixing ring around the fitting portions 30a and 30b.

The bulged portion 30c of the first balloon 30 fitted to the tip 36 is inflated into a substantially spherical shape by blowing air through the air supply intake 28 shown in Fig. 2. On the other hand, by sucking air through the air supply intake 28, the bulged portion 30c is contracted and adhered tightly to the outer circumferential face of the tip 36.

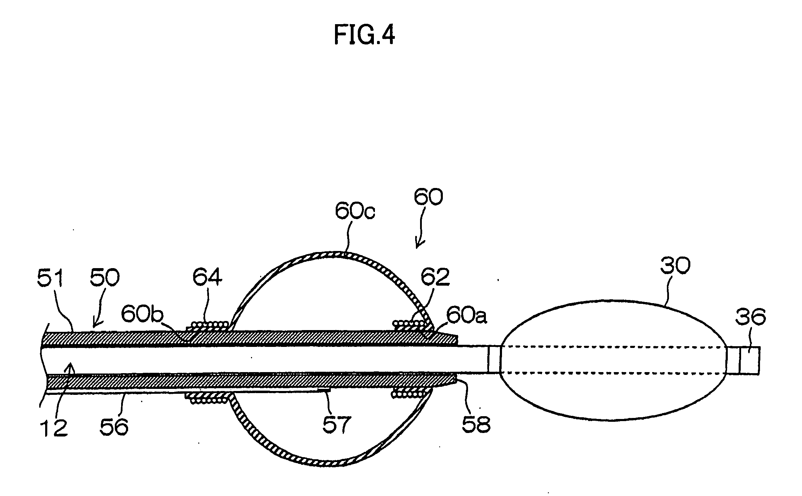

The over-tube 50 shown in Fig. 1 is formed of a tube body 51 and a grip 52. The tube body 51, formed in a tubular shape as shown in Fig. 4, has a slightly larger inner diameter than the outer diameter of the insert part 12. The insert part 12 of the endoscope 10 is inserted through the base end aperture 52A of the grip 52 shown in Fig. 1 into the tube body 51.

On the other hand, the tube body 51 is made of a flexible resin tube, consisting of urethane or the like as its base element, of which the outer circumferential face is coated with a hydrophilic material (lubricating coat) in a uniform thickness and the inner circumferential face is also coated with a hydrophilic material in a prescribed thickness. The material for the inner coating will be described in more detail afterwards. Applicable examples of the hydrophilic coating material include polyvinylpyrrolidone, acryl resin and silicone resin.

A balloon air supply port 54 is provided on the base end side of the tube body 51. An air supply tube 56 of about 1 mm in bore is connected to the balloon air supply port 54, and this tube 56, adhered to the outer circumferential face of the tube body 51, extends to the tip of the tube body 51.

The tip 58 of the tube body 51 is formed in a tapered shape as shown in Fig. 4. A second balloon 60 consisting of an elastic material, such as rubber, is fitted to the base end side of the tip 58 of the tube body 51. The second balloon 60, fitted in such a state that the tube body 51 penetrates it, is formed of a bulged portion 60c in the middle and fitting portions 60a and 60b on its two sides. The fitting portion 60a at the tip is folded back into the bulged portion 60c, and the folded fitting portion 60a is fixed to the tube body 51 by winding an X-ray contrast thread around it. The fitting portion 60b is arranged outside the second balloon 60 and fixed to the tube body 51 by winding a thread 64 around it.

The bulged portion 60c is formed to be substantially spherical in its natural state (neither inflated nor contracted) and to be greater than the first balloon 30 in its natural state (neither inflated nor contracted). Therefore, when air is supplied to the first balloon 30 and the second balloon 60 at the same pressure, the outer diameter of the bulged portion 60c of the second balloon becomes greater than that of the bulged portion 30c of the first balloon 30. The configuration is such that, if the outer diameter of the first balloon 30 is φ 25 mm for instance, the outer diameter of the second balloon 60 is φ 50 mm.

The tube 56 opens to the inside of the bulged portion 60c to form an air supply intake 57. Therefore, when air is supplied through the balloon air supply port 54, air is blown out of the air supply intake 57 to inflate the bulged portion 60c. Or when air is sucked through the balloon air supply port 54, air is sucked through the air supply intake 57 to contract the second balloon 60. Reference numeral 66 in Fig. 1 denotes an inlet for pouring lubricant, such as water, into the tube body 51.

On the other hand, the balloon control unit 100 is a device to supply and suck fluid, such as air, to and from the first balloon 30 as well as to supply and suck similar fluid to and from the second balloon 60. The balloon control unit 100 comprises a device body 102 provided with a pump, a sequencer and so forth (none shown) and a hand switch 104 for use in remote control.

A power switch SW1, a stop switch SW2, a pressure gauge 106 for the first balloon 30 and a pressure gauge 108 for the second balloon 60 are arranged on the front panel of the device body 102. Also, a tube 110 for supplying and sucking air to and from the first balloon 30 and a tube 120 for supplying and sucking air to and from the second balloon 60 are disposed on the front panel of the device body 102. Fluid reservoir tanks 130 and 140 are provided midway on the tubes 110 and 120 to hold any body fluid flowing back from the first balloon 30 and the second balloon 60 in the event that the first balloon 30 and/or the second balloon 60 become broken.

On the other hand, the hand switch 104 is provided with a stop switch SW3 similar to the stop switch SW2 on the device body 102 side, an ON/OFF switch SW4 for supporting the pressurization and depressurization of the first balloon 30, a pause switch SW5 for keeping the pressure of the first balloon 30, an ON/OFF switch SW6 for supporting the pressurization and depressurization of the second balloon 60, and a pause switch SW7 for keeping the pressure of the second balloon 60. This hand switch 104 is electrically connected to the device body 102 via a cable 150.

The balloon control unit 100 configured as described above supplies air to the first balloon 30 and the second balloon 60 to inflate them and keeps them in the inflated state by controlling the pressure of that air. Or it sucks air from the first balloon 30 and the second balloon 60 to contract them and keeps them in the contracted state by controlling the pressure of that air.

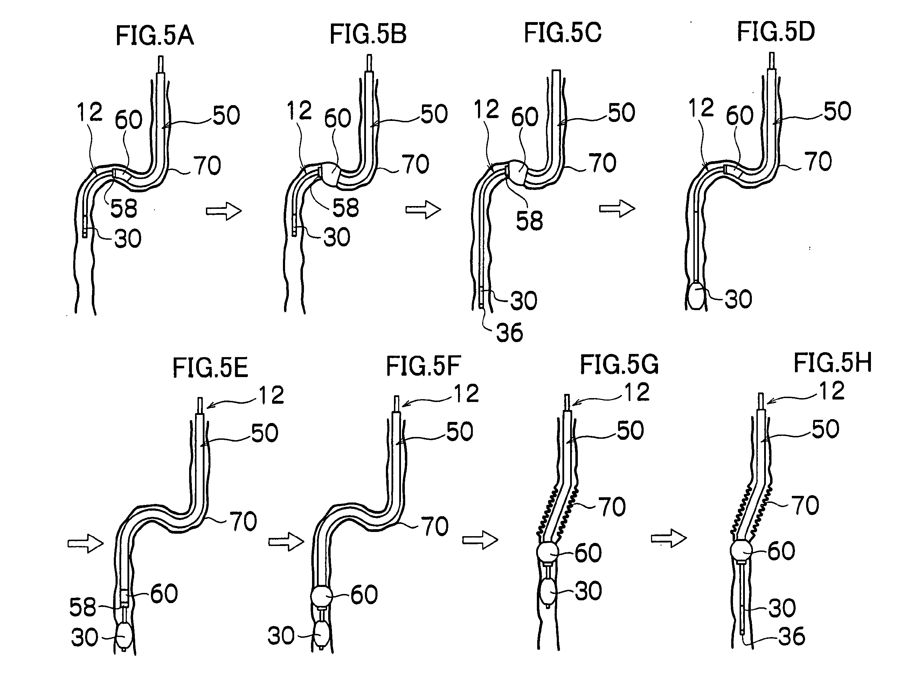

The method of operating the double-balloon type endoscope apparatus will now be described with reference to Figs. 5A through 5H.

First, as shown in Fig. 5A, the insert part 12 in a state of being covered by the over-tube 50 is inserted into an intestinal tract (e.g. the descending part of the duodenum) 70. In this process, the first balloon 30 and the second balloon 60 are kept contracted.

Then, as shown in Fig. 5B, in a state in which the tip 58 of the over-tube 50 is inserted into the bent portion of the intestinal tract 70, air is supplied to the second balloon 60 to inflate it. This causes the second balloon 60 to be engaged with the intestinal tract 70 and the tip 58 of the over-tube 50 to be fixed to the intestinal tract 70.

Next, as shown in Fig. 5C, only the insert part 12 of the endoscope 10 is inserted deep into the intestinal tract 70. Then, by inflating the first balloon 30 with air, the first balloon 30 is fixed to the intestinal tract 70 as shown in Fig. 5D. Since the inflated size of the first balloon 30 is smaller than that of the second balloon 60, the load on the intestinal tract 70 can be kept small to prevent the intestinal tract 70 from being damaged.

Then, after contracting the second balloon 60 by sucking air from it, the over-tube 50 is pressed in as shown in Fig. 5E and inserted along the insert part 12. After the tip 58 of the over-tube 50 is pressed in to a position close to the first balloon 30, the second balloon 60 is inflated by supplying air to it as shown in Fig. 5F. This causes the second balloon 60 to be fixed to the intestinal tract 70. Thus, the intestinal tract 70 is held by the second balloon 60.

Next, as shown in Fig. 5G, the over-tube 50 is hauled in. This causes the intestinal tract 70 to contract into a straight shape to eliminate any extra sagging or bending of the over-tube 50. Incidentally, whereas both the first balloon 30 and the second balloon 60 are engaged with the intestinal tract 70 when the over-tube 50 is hauled in, the frictional resistance of the first balloon 30 is smaller than that of the second balloon 60. Therefore, even if the first balloon 30 and the second balloon 60 move away from each other, the first balloon 30 with the smaller frictional resistance slides along the intestinal tract 70, and accordingly the intestinal tract 70 is prevented from being pulled by both balloons 30 and 60 and thereby damaged.

Then, as shown in Fig. 5H, the first balloon 30 is contracted by sucking air from it, and the tip 36 of the insert part 12 is inserted as deep as possible into the intestinal tract 70. Thus, the inserting operation shown in Fig. 5C is again performed. This enables the tip 36 of the insert part 12 to be inserted deep enough into the intestinal tract 70. If it is desired to insert the insert part 12 even deeper, the press-in operation shown in Fig. 5E can be performed after the fixing operation shown in Fig. 5D, followed by repetition of the holding shown in Fig. 5F, the hauling-in shown in Fig. 5G and the insertion shown in Fig. 5H in this sequence, which would enable the insert part 12 to be inserted into a further depth in the intestinal tract 70.

In the double-balloon type endoscope apparatus equipped with such an over-tube 50, the over-tube 50 in this embodiment of the invention is improved in the ease of insertion into the winding intestinal tract (in vivo) 70, and contributes to the durability of the hydrophilic coating material and accordingly to extending the service life of the tube.

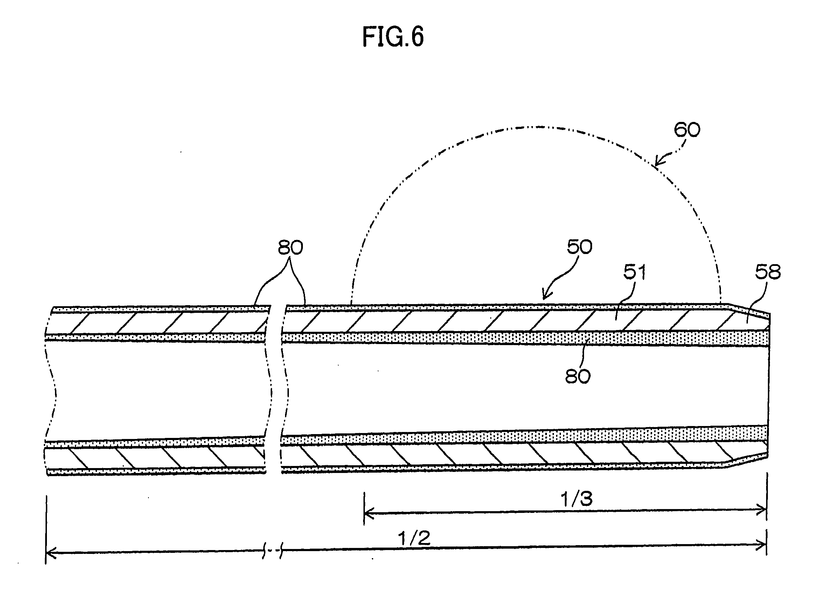

Thus, as shown in Fig. 6, in a resin tube (denoted hereby reference numeral 51, the same as the tube body 51 for the sake of convenience) 51 which is the base material of the over-tube 50, a hydrophilic coating material 80 to cover its inner circumferential face is formed into a coat whose thickness gradually increase from the base end side toward the tip 58. This enables the hydrophilic coat 80 near and including the tip 58 to wear longer and remain usable for a longer period.

As shown in Fig. 6, the length of the variable-thickness portion, from the tip 58 toward the base end, of the hydrophilic coating material 80 is from about 1/3 to about 1/2 of the overall length of the resin tube 51. The tip of the over-tube 50 is more worn than its base end as it is subject to friction with the tip 36 of the insert part 12 of the endoscope that is inserted. For this reason, the tip and its vicinities are covered with more layers of the hydrophilic coat 80 than other parts are. As multi-layered application of the hydrophilic coating material 80 over the full length of the over-tube 50 would make the hydrophilic coat 80 thicker even where no extra thickness is required, only about 1/3 to about 1/2 of the overall length from the tip, the part more subject to bending when the tube is inserted into a body cavity is made thicker. This is because the part more subject to bending is more subject to friction with the tip 36 of the insert part 12 of the endoscope. The thickness of the hydrophilic coating material 80 can be varied merely by drying, after coating the resin tube 51 with the hydrophilic coating material 80, the resin tube 51 with its tip 58 down. Thus, the drying of the hydrophilic coating material 80 while it is allowed to sag down by its own weight makes its thickness increase toward the tip 58. A similar effect can be achieved by gradually increasing the thickness of the hydrophilic coating material from the base end toward the tip 58.

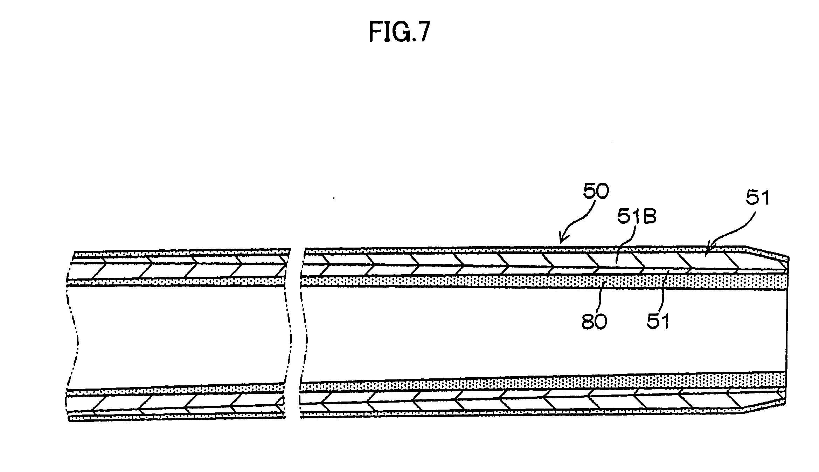

Fig. 7 is a sectional view showing another example of over-tube 50 in Fig. 6. The resin tube 51 of the over-tube 50 shown in Fig. 7 comprises two kinds of members, including a hard member 51A inside and a soft member 51B outside. The thickness of the hard member 51A is less toward the tip 58 than on the base side, and the reverse is true of the soft member 51B, with the result that the wall thickness of the over-tube 50 is uniformized over its full length by so positioning the soft member 51B as to meet the outer face of the hard member 51A. This contributes to increasing the flexibility of the over-tube 50 toward the tip 58.

Since the reaction forces at the tip 58 and its vicinities are thereby reduced, the frictional resistance with the curved living body is decreased and the insertion of the over-tube 50 into the living body is made easier. As the hydrophilic material 80 which coats the inner circumferential face of the hard member 51A is made thicker at the tip 58 than toward the base end as in the configuration shown in Fig. 6, the hydrophilic coating material 80 wears better and can remain useful for a longer period. Further, by arranging the hard member 51 A inside, the intrusion of the tip 36 of the insert part 12 of the endoscope into the inner face of the over-tube 50 is reduced compared with a configuration in which the inner face consists of a soft member, the insert part of the endoscope 12 is made even easier to insert.

Although the embodiment described above uses the over-tube 50 as the insertion aid, this is not the only available option, but a sliding tube for transanal insertion can be used as well.

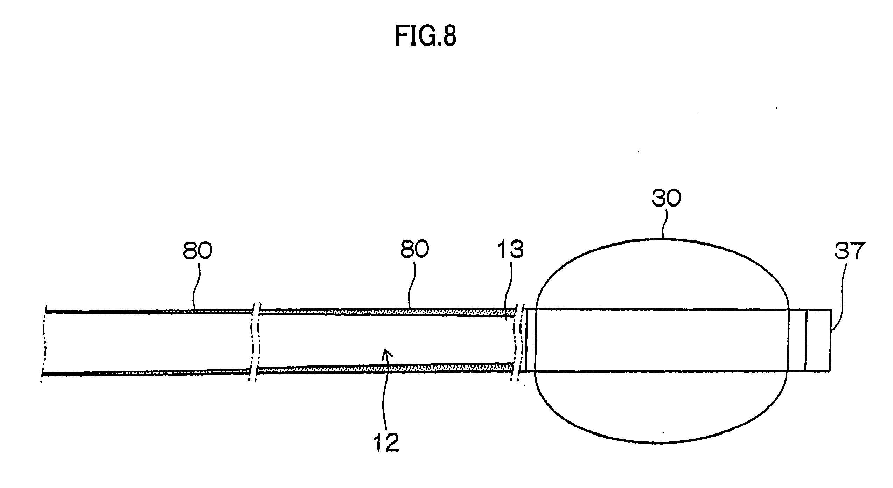

Fig. 8 is a partial sectional view of an example in which the outer circumferential face of the insert part 12 of the endoscope is coated with the hydrophilic material 80, and the thickness of the hydrophilic coat 80 is gradually increased from the base end (the end linked to the manipulating part 14 in Fig. 1) toward the tip (the end where the first balloon 30 is fitted) 13 of the insert part.

By making the thickness of the hydrophilic material 80 coating the outer circumferential face of the insert part of the endoscope 12 greater toward the tip 13 in the inserting direction of the insert part of the endoscope 12 than on the base end side in the inserting direction in this way, the hydrophilic coating material 80 is enabled to wear better and remain useful for a longer period. Combined use of the insert part 12 of the endoscope coating with the hydrophilic material 80 and the over-tube 50 coated with the hydrophilic material 80 shown in Fig. 6 and Fig. 7 would enable both lubricating coats 80 to wear even longer.

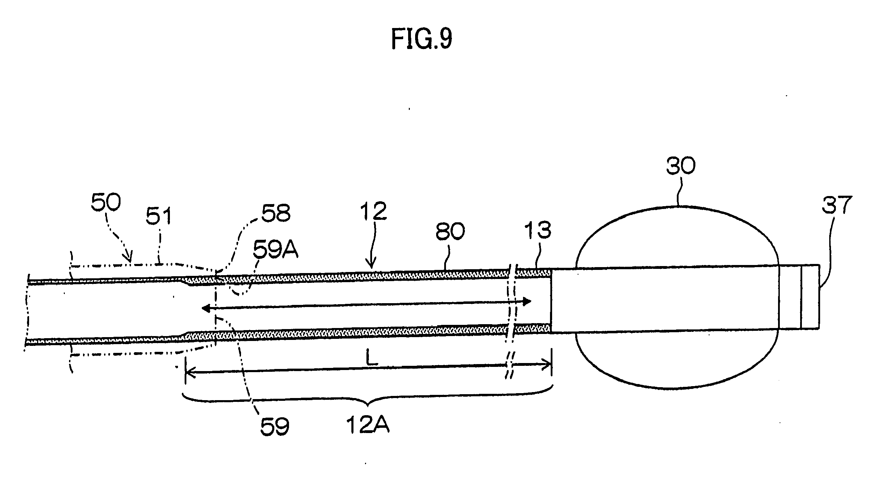

Fig. 9 is a partial sectional view of an example in which the thickness of the hydrophilic coating material 80 is increased only in the tip portion of the insert part 12 of the endoscope. In this drawing, the thicker portion of the hydrophilic coat 80 is the tip portion in the inserting direction of the insert part 12 of the endoscope, the length L portion 12A which is inserted into and withdrawn out of the tip 58 of the over-tube 50. In the insert part 12, this length L portion 12A is in sliding contact with an inner circumferential corner 59A of an aperture 59 formed in the tip 58 of the over-tube 50, and is subject to a force in the direction of peeling the hydrophilic coating material 80 off. Therefore, by making the hydrophilic material 80 coating at least the length L portion 12A thicker than elsewhere, the object of the invention under this application to enable the hydrophilic coating material 80 wear longer and remain useful for a longer period can be achieved.

On the other hand, whereas the outer circumferential face of the over-tube 50 shown in Fig. 6 is also coated with the lubricating material 80, the friction coefficient of this coated outer circumferential face of the over-tube 50 is set to be 5 to 30% of the friction coefficient of the surface, not coated with the lubricating material, of the second balloon 60. In this embodiment of the invention, the lubricating material 80 coating the over-tube 50 is polyvinylpyrrolidone (0.08 in friction coefficient µ), corresponding to 16% of that of the second balloon 60, which is made of natural rubber (0.5 in friction coefficient µ).

Incidentally, since the double-balloon type endoscope apparatus having the first balloon 30 and the second balloon 60 is frequently pressed into a body cavity, it is essential to reduce the frictional resistance of the circumferential face of the over-tube 50 which is in contact with the wall of the body cavity it enters into. On the other hand, it is no less important to secure friction with the wall of the body cavity to ensure a sufficient holding force when the second balloon 60 is inflated and adheres closely to the wall of the body cavity.

In view of this background, the frictional resistance to the wall of the body cavity is reduced, and at the same time an appropriate level of frictional resistance is prescribed for the second balloon 60 for which a relatively high level of frictional resistance is secured, by coating the outer circumferential face of the over-tube 50 with the lubricating material. Thus, the frictional resistance of the outer circumferential face of the over-tube 50 coated with the lubricating material is set to be 5 to 30% of the frictional resistance of the second balloon 60. If the proportion of that frictional resistance is less than 5%, the over-tube 50 will prove too slippery in the hand of the operator manipulating it, and this might invite mishandling. Or if the proportion is more than 30%, it will become difficult to insert or withdraw the over-tube 50 against the wall of the body cavity. Therefore, by setting the proportion of frictional resistance between 5 and 30%, it is made possible to insert and withdraw the over-tube 50 with satisfactory ease. By setting the proportion between 12 and 16%, the ease of inserting and withdrawing the over-tube 50 into and out of the body cavity can be made even more satisfactory.

To repeat an earlier description, the over-tube 50 of the double-balloon type endoscope apparatus which repeats reciprocating movement relative to the intestinal tract (body cavity) 70 as shown in Fig. 5 is required to be, in addition to being able to smoothly slide along the insert part 12 of the endoscope, (1) able to smoothly slide along the inner wall of the body cavity, (2) adequately flexible, and (3) adequately resilient.

Whereas examples of materials for possible use as the base of the over-tube 50 include polytetrafluoroethylene, polyurethane, vinyl chloride and synthetic rubber, polytetrafluoroethylene does not meet the requirement of (2) though it does satisfy those of (1) and (3). Polyurethane and vinyl chloride, though meeting the requirements of (2) and (3), fails to meet that of (1). Synthetic rubber satisfies the requirement of (2) but not those of (1) and (3). In view of these different characteristics, it is considered that the requirement of (1) can be met by coating the outer circumferential face of the over-tube 50 with a lubricating material and selecting polyurethane and vinyl chloride satisfying the requirements of (2) and (3) as the base materials for the over-tube 50.

On the other hand, the requirements for the lubricating coat material include (A) sufficient sliding ease and (B) good compatibility with the base material.

Examples of materials for possible use as the lubricating coat material include polyvinylpyrrolidone, Xylocaine jelly and olive oil. Polyvinylpyrrolidone particularly well satisfies the requirement of (A) and, regarding that of (B), is compatible with polyurethane but less so with vinyl chloride. Xylocaine jelly cannot satisfy the requirement of (A) though it is well compatible with both polyurethane and vinyl chloride regarding that of (B). Similarly, olive oil cannot meet the requirement of (A) though it is well compatible with both polyurethane and vinyl chloride regarding that of (B).

On the basis of these findings, the over-tube 50 in this embodiment uses a polyurethane base coated with polyvinylpyrrolidone.

Further, the outer circumferential face of the insert part 12 of the endoscope is coated with the hydrophilic material 80, and the friction coefficient of this outer circumferential face is set to 5 to 30% of the friction coefficient of the first balloon 30, for which a relatively high level of frictional resistance is secured. If the proportion of that frictional resistance is less than 5%, the over-tube 50 will prove too slippery in the hand of the operator manipulating it, and this might invite mishandling. Or if the proportion is more than 30%, it will become difficult to insert or withdraw the insert part 12 of the endoscope against the wall of the body cavity. Therefore, by setting the proportion of frictional resistance between 5 and 30%, it is made possible to insert and withdraw the insert part 12 of the endoscope with satisfactory ease. By setting the proportion between 12 and 16%, the ease of inserting and withdrawing the insert part 12 of the endoscope into and out of the body cavity can be made even more satisfactory.

Although the endoscope apparatus embodying the invention has been described so far with reference to a double-balloon type endoscope apparatus, it is also preferable to prescribe for the outer circumferential face of an individual over-tube 50 having a balloon or of the insert part 12 of an endoscope having a balloon a frictional resistance corresponding to 5 to 30% of that of the balloon.

## Claims

1. An endoscope apparatus provided with an insertion aid (50) through whose base end an insert part (12) of the endoscope (10) is inserted and whose tip (58) is fitted with a balloon (30), the balloon (30) being closely adhered to a body cavity by being inflated, **characterized in that**:
the outer circumferential face of the insertion aid (50) is coated with a lubricating coat material, and the friction coefficient of the outer circumferential face of the insertion aid (50) is set to 5 to 30% of the friction coefficient of the balloon (30).

2. An endoscope apparatus provided with an endoscope the tip (58) of whose insert part (12) is fitted with a balloon (30), the balloon (30) being closely adhered to a body cavity by being inflated, **characterized in that**:
the outer circumferential face of the insert part (12) of the endoscope (10) is coated with a lubricating coat material, and the friction coefficient of the outer circumferential face of the insert part (12) is set to 5 to 30% of the friction coefficient of the balloon (30).

3. An endoscope apparatus provided with an endoscope the tip (58) of whose insert part (12) is fitted with a first balloon (30) and an insertion aid (50) which covers the insert part (12) to guide the insertion of the insert part (12) and whose tip is fitted with a second balloon (60), the first balloon (30) and the second balloon (60) being closely adhered to a body cavity by being inflated, **characterized in that**:
the outer circumferential face of the insertion aid (50) is coated with a lubricating coat material, and the friction coefficient of the outer circumferential face of the insertion aid (50) is set to 5 to 30% of the friction coefficient of the second balloon (60).

4. The endoscope apparatus according to claim 3, wherein the outer circumferential face of the insert part (12) of the endoscope (10) is coated with a lubricating coat material, and the friction coefficient of the outer circumferential face of the insert part (12) is set to 5 to 30% of the friction coefficient of the first balloon (30).
